(19) Europäisches Patentamt
European Patent Office
Office européen des brevets

(11) **EP 4 684 777 A1**

(12) **EUROPEAN PATENT APPLICATION**

(43) Date of publication:
**28.01.2026 Bulletin 2026/05**

(21) Application number: **24386091.3**

(22) Date of filing: **24.07.2024**

(51) International Patent Classification (IPC):
***A61K 9/20*** *(2006.01)* ***A61K 31/4985*** *(2006.01)*
***A61K 31/519*** *(2006.01)*

(52) Cooperative Patent Classification (CPC):
(C-Sets available)
**A61K 9/2077; A61K 31/4985; A61K 31/505;**
A61K 9/2013; A61K 9/2018; A61K 9/2054 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Adalvo Limited**
  **San Gwann, SGN 3000 (MT)**
- **Win Medica S.A.**
  **15123 Marousi, Attica (GR)**

(72) Inventors:
- **Zerrin, Ozge Samuk**
  **Istanbul (TR)**
- **Bayrak, Gökhan**
  **34535 Büyükçekmece, Istanbul (TR)**
- **Erdogan, Akif**
  **Tekirdag (TR)**
- **Sezer, Dilay**
  **Tekirdag (TR)**
- **Marasli, Mustafa**
  **Tekirdag (TR)**

(74) Representative: **Slavík, Jiri**
**Artistu 3183/14**
**193 00 Praha-Horní Pocernice (CZ)**

(54) **A FIXED-DOSE PHARMACEUTICAL COMPOSITION COMPRISING (2S)-2-[(4,6-DIMETHYLPYRIMIDIN-2-YL)OXY]-3-METHOXY-3,3-DIPHENYLPROPANOIC ACID AND (6R,12AR)-6-(1,3-BENZODIOXOL-5-YL)-2-METHYL-2,3,6,7,12,12A-HEXAHYDROPYRAZINO {1',2:1,6]PYRIDO[3,4-B]INDOLE-1,4-DIONE**

(57) The present invention relates to a fixed-dose pharmaceutical composition comprising (2S)-2-[(4,6-dimethylpyrimidin-2-yl)oxy]-3-methoxy-3,3-diphenylpropanoic acid or its pharmaceutically acceptable salt, ester, solvate, polymorph, enantiomer or mixture thereof and (6R,12aR)-6-(1,3-benzodioxol-5-yl)-2-methyl-2,3,6,7,12,12a-hexahydropyrazino[1',2':1,6]pyrido[3,4-b]in dole-1,4-dione or its pharmaceutically acceptable salt, ester, solvate, polymorph, enantiomer or mixture thereof, wherein the composition is further comprising one or more pharmaceutically acceptable excipients, and also a process for the preparation thereof.

EP 4 684 777 A1

(52) Cooperative Patent Classification (CPC): (Cont.)

C-Sets
**A61K 31/4985, A61K 2300/00;**
**A61K 31/505, A61K 2300/00**

## Description

### Technical Field

The present invention relates to a fixed-dose pharmaceutical composition comprising (2S)--2-[(4,6-dimethylpyrimidin-2-yl)oxy]-3-methoxy-3,3-diphenylpropanoic acid, also known as Ambrisentan, or its pharmaceutically acceptable salt, ester, solvate, polymorph, enantiomer or mixture thereof and (6R,12aR)-6-(1,3-benzodioxol-5-yl)-2-methyl-2,3,6,7,12,12a-hexahydropyrazino[1',2':1,6]pyrido[3,4-b]in dole-1,4-dione, also known as Tadalafil, or its pharmaceutically acceptable salt, ester, solvate, polymorph, enantiomer or mixture thereof, wherein the composition is further comprising one or more pharmaceutically acceptable excipients, and also a process for the preparation thereof.

### Background Art

WO9425442, WO9526716 and WO9611914 disclose the compound (2S)-2-[(4,6-dimethylpyrimidin-2--yl) oxy]-3-methoxy-3,3-diphenylpropanoic acid of formula I, also known as Ambrisentan, a process for the preparation thereof, and its use in the treatment of hypertension and pulmonary hypertension.

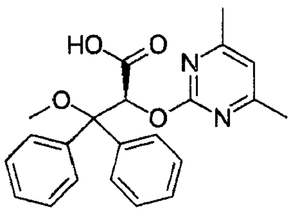

**(I)**

Ambrisentan is an endothelin receptor antagonist and is selective for the type A endothelin receptor (ETA). It is marketed under the brand names Volibris and Letairis (2.5, 5 and 10 mg strengths) and is used in the treatment of pulmonary arterial hypertension (PAH).

WO9519978, WO9703985 and WO0015228 disclose the compound (6R,12aR)-6-(1,3-benzodioxol-5-yl)--2-methyl-2,3,6,7,12,12a-hexahydropyrazino[1',2':1,6]pyrido[3,4-b]indole-1,4-dione of formula II, also known as Tadalafil, a process for the preparation thereof, and its use in the treatment of erectile dysfunction, benign prostatic hyperplasia (BPH) and pulmonary hypertension.

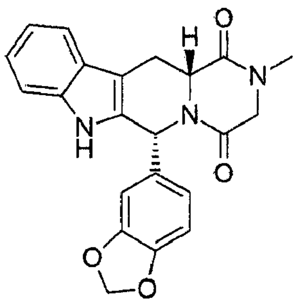

**(II)**

Tadalafil is classified as a phosphodiesterase-5 (PDE-5) inhibitor. It is marketed under the brand name Cialis (2.5, 5, 10 and 20 mg strengths) and is used in the treatment of erectile dysfunction and benign prostatic hyperplasia (BPH). Tadalafil is also marketed under the brand name Adcirca (20 mg strength) and is used in the treatment of pulmonary arterial hypertension (PAH).

WO2008073928 discloses the use of Ambrisentan in the treatment of pulmonary arterial hypertension (PAH), optionally in combination with a phosphodiesterase-5 (PDE-5) inhibitors such as sildenafil, tadalafil and vardenafil.

WO2012051559 discloses a synergistic effect of Ambrisentan, when administered in combination with Tadalafil, for the treatment of pulmonary arterial hypertension (PAH). No examples of a fixed-dose pharmaceutical composition of Ambrisentan and Tadalafil, even in form of a multi-layered dosage form are described therein.

Furthermore, co-administration of separate preparations of Ambrisentan and Tadalafil has several disadvantages, including reduced patient compliance, increased complexity and pill burden for patients, often with further

concomitant drugs being prescribed to the patient.

Therefore, there remains a need to develop a bioequivalent, simple, reproducible, cost-effective, and feasible fixed-dose pharmaceutical composition comprising Ambrisentan and Tadalafil and a suitable manufacturing process thereof, that is able to provide the desired dissolution and disintegration properties of both active ingredients Ambrisentan and Tadalafil during release as well as during the shelf-life period of the pharmaceutical composition. At the same time, this would ensure, that these properties are comparable to the reference drug mono-products comprising respectively Ambrisentan and Tadalafil, so that patients currently treated with the respective separate preparations may be easily and safely transitioned to a fixed-dose composition comprising both active ingredients, having all above-mentioned benefits. There is hence a need to provide a bioequivalent pharmaceutical product having the desired stability during release as well as during the shelf-life period of the pharmaceutical composition.

Ambrisentan and Tadalafil show several incompatibilities when formulated together (for example, increased amount of impurities, unfavourable dissolution times, chemical instability, etc.), especially when Tadalafil is present in an excess ratio in the pharmaceutical composition compared to Ambrisentan, as it normally required with respect to the dosing of both active ingredients. Therefore, the production of any such feasible fixed-dose pharmaceutical composition is expected to face extensive difficulties, which would make such production cumbersome and expensive. It is generally assumed that, at best, a multi-layer solid oral dosage form could provide a fixed-dose composition of Ambrisentan and Tadalafil, but even such formulation has not yet been developed and marketed, despite the rather long period during which the benefits of the combined effect of these two active ingredients have been widely known. At most, a combination pack comprising separate dosage forms of Ambrisentan and Tadalafil in a combined blister has been developed throughout the years, which however does not address at all the disadvantages of having separate dosage forms as pointed out above.

It is therefore the object of this invention to solve problems of the prior art and to provide a pharmaceutical composition comprising both Ambrisentan and Tadalafil that addresses the drawbacks identified in the prior art.

## Disclosure of the Invention

The inventors have surprisingly found that a fixed-dose pharmaceutical composition comprising (2S)-2-[(4,6-dimethylpyrimidin-2-yl)oxy]-3-methoxy-3,3-diphenylpropanoic acid, also known as Ambrisentan, or its pharmaceutically acceptable salt, ester, solvate, polymorph, enantiomer or mixture thereof and (6R,12aR)-6-(1,3-benzodioxol-5-yl)-2-methyl--2,3,6,7,12,12a-hexahydropyrazino[1',2':1,6]pyrido[3,4-b]indole-1,4-dione, also known as Tadalafil, or its pharmaceutically acceptable salt, ester, solvate, polymorph, enantiomer or mixture thereof, may be provided as a one-layer solid oral dosage form, and that there is no need for a double or a multi-layer solid oral dosage form to provide this fixed-dose pharmaceutical composition with the desired properties, despite the incompatibility issues between the two active ingredients Ambrisentan and Tadalafil. The present inventors have thus surprisingly found that, despite said technical prejudice, resulting from incompatibility of the two active ingredients Ambrisentan and Tadalafil, which would lead to a double and/or multi-layered solid oral dosage form of Ambrisentan and Tadalafil in order to address the incompatibility issues, the fixed-dose pharmaceutical composition according to the present invention as defined in the claims, provides a bioequivalent pharmaceutical composition, having the desired stability during release, as well as during the shelf-life period of the pharmaceutical composition.

The fixed-dose pharmaceutical composition according to the present invention is comprising (2S)-2-[(4,6-dimethylpyrimidin-2-yl)oxy]-3-methoxy-3,3-diphenylpropanoic acid, also known as Ambrisentan, or its pharmaceutically acceptable salt, ester, solvate, polymorph, enantiomer or mixture thereof and (6R,12aR)-6-(1,3-benzodioxol-5-yl)-2-methyl-2,3,6,7,12,12a-hexahydropyrazino[1',2':1,6]pyrido[3,4-b]in dole-1,4-dione, also known as Tadalafil, or its pharmaceutically acceptable salt, ester, solvate, polymorph, enantiomer or mixture thereof with one or more pharmaceutically acceptable excipients, and is provided as a one-layer solid oral dosage form. In a preferred embodiment the solid oral dosage form is in a form of a tablet for oral administration. Preferably the tablet is an immediate release tablet and even more preferably, it is an immediate release film-coated tablet.

In a preferred embodiment, the weight ratio of Ambrisentan and Tadalafil in the fixed-dose pharmaceutical composition according to the present invention is from about 1:4 to about 1:8.

In a preferred embodiment, the fixed-dose pharmaceutical composition according to the present invention is also able to provide desired dissolution and disintegration properties for both active ingredients Ambrisentan and Tadalafil during release, as well as during the shelf-life period of the pharmaceutical composition.

In a preferred embodiment, the fixed-dose pharmaceutical composition has a disintegration time of less than 5 minutes, preferably less than 4 minutes, as measured by European Pharmacopeia 11.0, monograph 2.9.1. - Test A, in distilled water without using the disc, during initial release, as well as after 3 and/or after 6 months at accelerated conditions at 40°C $\pm$ 2°C and 75% RH $\pm$ 5% RH.

In a preferred embodiment, the fixed-dose pharmaceutical composition has a dissolution profile of Ambrisentan or its pharmaceutically acceptable salt, ester, solvate, polymorph, enantiomer or mixture thereof, such that within the first 30 minutes, preferably within the first 15 minutes, more than 85% of Ambrisentan, or its pharmaceutically acceptable salt,

ester, solvate, polymorph, enantiomer or mixture thereof, is released, as measured by European Pharmacopeia 11.0, monograph 2.9.3., dissolution Apparatus 2 in 900 ml of pH 5.0 acetate buffer solution at 37°C ± 0.5°C with the paddle speed of 60 rpm, during initial release, as well as after 3 and/or after 6 months either at normal conditions (25°C ± 2°C and 65% RH ± 5% RH) or at accelerated conditions (40°C ± 2°C and 75% RH ± 5% RH).

For the fixed-dose pharmaceutical composition, the desired dissolution profile of Tadalafil or its pharmaceutically acceptable salt, ester, solvate, polymorph, enantiomer or mixture thereof, is such that within the first 30 minutes, preferably within the first 15 minutes, more than 85% of Tadalafil, or its pharmaceutically acceptable salt, ester, solvate, polymorph, enantiomer or mixture thereof, is released, as measured by as measured by European Pharmacopeia 11.0, monograph 2.9.3., dissolution Apparatus 2 in 1000 ml of 0.4% sodium lauryl sulfate solution at 37°C ± 0.5°C with the paddle speed of 50 rpm, during initial release, as well as after 3 and/or after 6 months either at normal conditions (25°C ± 2°C and 65% RH ± 5% RH) or at accelerated conditions (40°C ± 2°C and 75% RH ± 5% RH).

The fixed-dose pharmaceutical composition according to the present invention preferably comprises Tadalafil granulate and Ambrisentan granulate, wherein preferably the granulates are present as separate granules and are granulated separately. In a preferred embodiment of the fixed-dose pharmaceutical composition, the Tadalafil granulate further comprises excipients selected from a diluent, a disintegrant, a binder, a surfactant and/or a mixture thereof. In a preferred embodiment the fixed-dose pharmaceutical composition, the Ambrisentan granulate further comprises excipients selected from a diluent, a binder and/or a mixture thereof. Preferably, an extra-granular lubricant is present in the pharmaceutical composition as an extra-granular excipient.

In a preferred embodiment, the Ambrisentan granulate is free from any lubricant and/or any disintegrant and/or the Tadalafil granulate is free from any lubricant. Such composition further supports the preferred dissolution properties and disintegration of the two active ingredients Ambrisentan and Tadalafil in the fixed-dose pharmaceutical composition according to the invention, as defined in the claims.

The inventors have further surprisingly found that the fixed-dose pharmaceutical composition according to the present invention, when prepared by a process comprising fluid-bed granulation of the Tadalafil granulate and a high-shear mixer wet granulation of the Ambrisentan granulate, provides the preferred dissolution properties and disintegration time for both active ingredients Ambrisentan and Tadalafil initially at release and as well as during the shelf-life period of the pharmaceutical composition.

When the fixed-dose pharmaceutical composition is prepared by a high-shear mixer wet granulation of both Ambrisentan granulate and Tadalafil granulate, which is the standard technique in the pharmaceutical industry and the first choice for BCS (biopharmaceutical classification system) class II compounds, such as Ambrisentan and Tadalafil, the dissolution properties and disintegration time of the fixed-dose pharmaceutical composition exhibit a significant undesired increase under the accelerated conditions (40°C and 75% RH). This was even more surprising, since this increase in disintegration time was not further accompanied by a change in the hardness and/or degradation of the fixed-dose pharmaceutical composition either at normal conditions (25°C ± 2°C and 65% RH ± 5% RH) or at accelerated conditions (40°C ± 2°C and 75% RH ± 5% RH) for the same qualitative and quantitative pharmaceutical composition, which would normally be expected.

In another preferred embodiment, the Ambrisentan granulate of the fixed-dose pharmaceutical composition according to the present invention, comprises:

a) from about 1% wt. to about 5% wt. of Ambrisentan, or its pharmaceutically acceptable salt, ester, solvate, polymorph, enantiomer or mixture thereof, with respect to the core solid dosage form weight;

b) from about 0.5% wt. to about 2% wt. of binder with respect to the core solid dosage form weight, where preferably the binder is selected from the group comprising pregelatinized starch (PGS), cellulose, microcrystalline cellulose, silicified microcrystalline cellulose, methyl cellulose, ethyl cellulose, hydroxypropyl methyl cellulose (HPMC), hydroxypropyl cellulose (HPC), sodium carboxy methyl cellulose, polyvinyl pyrrolidone (PVP), polyethylene glycol (PEG), polyvinyl alcohol/polyethylene glycol graft copolymer, polyvinyl alcohols, polymethacrylates, polyvinylcaprolactam, vinylpyrrolidone-vinyl acetate copolymers, and/or mixtures or derivatives thereof, more preferably, the binder is hydroxypropyl cellulose;

c) from about 40% wt. to about 55% wt. of diluent with respect to the core solid dosage form weight, where preferably the diluent is selected from the group comprising lactose anhydrous, lactose monohydrate, maltose, microcrystalline cellulose, mannitol, sorbitol and/or mixtures or derivatives thereof, more preferably, the diluent is mixture of lactose monohydrate and microcrystalline cellulose.

In another preferred embodiment, the Tadalafil granulate of the fixed-dose pharmaceutical composition according to the present invention, comprises:

a) from about 5% wt. to about 15% wt. of Tadalafil, or its pharmaceutically acceptable salt, ester, solvate, polymorph, enantiomer or mixture thereof, with respect to the core solid dosage form weight;

b) from about 30% wt. to about 35% wt. of diluent with respect to the core solid dosage form weight, where preferably the diluent is selected from the group comprising lactose anhydrous, lactose monohydrate, maltose, microcrystalline cellulose, mannitol, sorbitol and/or mixtures or derivatives thereof, more preferably, the diluent is lactose monohydrate;

c) from about 0.5% wt. to about 2% wt. of binder with respect to the core solid dosage form weight, where preferably the binder is selected from the group comprising pregelatinized starch (PGS), cellulose, microcrystalline cellulose, silicified microcrystalline cellulose, methyl cellulose, ethyl cellulose, hydroxypropyl methyl cellulose (HPMC), hydroxypropyl cellulose (HPC), sodium carboxy methyl cellulose, polyvinyl pyrrolidone (PVP), polyethylene glycol (PEG), polyvinyl alcohol/polyethylene glycol graft copolymer, polyvinyl alcohols, polymethacrylates, polyvinylcaprolactam, vinylpyrrolidone-vinyl acetate copolymers, and/or mixtures or derivatives thereof, more preferably, the binder is hydroxypropyl cellulose;

d) from about 1 to about 10% wt. of disintegrant with respect to the core solid dosage form weight, where preferably the disintegrant is selected from the group comprising croscarmellose sodium, carboxymethyl cellulose calcium, crospovidone, potassium, sodium starch glycolate, starch, pregelatinized starch and/or mixtures or derivatives thereof, more preferably, the disintegrant is croscarmellose sodium;

e) from about 0.1% to about 2% wt. of surfactant with respect to the core solid dosage form weight, where preferably the surfactant is selected from the group comprising sodium lauryl sulfate, polyoxyethylene sorbitan fatty acid esters (polysorbates) and/or mixtures or derivatives thereof, more preferably, the surfactant is sodium lauryl sulfate.

In a preferred embodiment, the fixed-dose pharmaceutical composition according to the present invention comprises Ambrisentan granulate and Tadalafil granulate in a weight ratio from about 1:0.5 to about 1:1.5, more preferably from about 1:0.7 to about 1:1, even more preferably from about 1:0.8 to about 1:0.9. This preferred weight ratio of Ambrisentan granulate and Tadalafil granulate provides and further supports the preferred dissolution and disintegration properties of both active ingredients Ambrisentan and Tadalafil during release as well as during the shelf-life period of the pharmaceutical composition.

Another aspect of the present invention is a process for preparation of the fixed-dose pharmaceutical composition according to the present invention, wherein the Tadalafil granulate is prepared by a fluid-bed granulation process and wherein the Ambrisentan granulate is prepared by a comprising high-shear mixer wet granulation process.

Preferably, the process for preparation of the fixed-dose pharmaceutical composition according to the present invention, is further comprising mixing of the Tadalafil granulate and the Ambrisentan granulate, addition of an extra granular lubricant, wherein preferably the extra granular lubricant is magnesium stearate, mixing the extra granular lubricant with the Tadalafil granulate and the Ambrisentan granulate, and further compression of the mixture in order to form a one-layer solid oral dosage form.

Preferably, the process for preparation of the Tadalafil granulate of the fixed-dose pharmaceutical composition according to the present invention, comprises: i) preparation of a mixture of the diluent, wherein preferably the diluent is lactose monohydrate, and the disintegrant, wherein preferably the disintegrant is croscarmellose sodium, ii) preparation of a granulation liquid by dissolving the binder in water, wherein preferably the binder is hydroxypropyl cellulose, and addition of the surfactant, wherein preferably the surfactant is sodium lauryl sulfate and addition of Tadalafil, and iii) fluid bed granulation by spraying the powder mixture with the granulation liquid.

Preferably, the process for preparation of the Ambrisentan granulate of the fixed-dose pharmaceutical composition according to the present invention, comprises: i) preparation of a mixture of Ambrisentan, the diluent(s), wherein preferably the diluent(s) is lactose monohydrate and/or microcrystalline cellulose and/or a mixture thereof, and one half of the total amount of the binder, wherein preferably the binder is hydroxypropyl cellulose, ii) preparation of a granulation liquid by dissolving the second half of the total amount of the binder in water, and iii) wet granulation of the powder mixture with the granulation liquid. The present invention further provides a fixed-dose pharmaceutical formulation of Ambrisentan and Tadalafil comprising the Ambrisentan granulate and the Tadalafil granulate as described herein above, obtainable by a process in which the Tadalafil granulate is prepared by a process comprising fluid-bed granulation and wherein the Ambrisentan granulate is prepared by a process comprising high-shear mixer wet granulation. Preferably, subsequently the granulates are mixed, an extra-granular lubricant is added, and the mixture is compressed into a solid oral dosage form, preferably into tablets.

In another preferred embodiment, the fixed-dose pharmaceutical composition according to the invention, wherein preferably said pharmaceutical composition is comprising 5 mg Ambrisentan and 20 mg Tadalafil and wherein in human in vivo pharmacokinetic studies in which said pharmaceutical composition is administered in fed state, said pharmaceutical composition preferably provides the following pharmacokinetic parameter selected from the group consisting of

- Ambrisentan maximum plasma concentration $C_{max}$ of about 440 ng/mL $\pm$ 10%,
- Ambrisentan area under the plasma concentration-time curve from 0 hours to the 72 hours $AUC_{0-t}$ of about 4584 hr-ng/ml ± 10%,

- Tadalafil maximum plasma concentration $C_{max}$ of about 404 ng/mL ± 10%,
- Tadalafil area under the plasma concentration-time curve from 0 hours to the 72 hours $AUC_{0-t}$ of about 13017 hr-ng/ml ± 10%.

In another preferred embodiment, the fixed-dose pharmaceutical composition according to the invention, wherein preferably the said pharmaceutical composition is comprising 10 mg Ambrisentan and 40 mg Tadalafil and wherein in human in vivo pharmacokinetic studies in which said pharmaceutical composition is administered in fasting state, said pharmaceutical composition preferably provides the following pharmacokinetic parameter selected from the group consisting of

- Ambrisentan maximum plasma concentration $C_{max}$ of about 1167 ng/mL ± 10%,
- Ambrisentan area under the plasma concentration-time curve from 0 hours to the 72 hours $AUC_{0-t}$ of about 9350 hr-ng/ml ± 10%,
- Tadalafil maximum plasma concentration $C_{max}$ of about 542 ng/mL ± 10%,
- Tadalafil area under the plasma concentration-time curve from 0 hours to the 72 hours $AUC_{0-t}$ of about 19624 hr-ng/ml ± 10%.

The term "binder", "binding agent" or "binding agent" as used herein is defined as an agent able to bind particles which cannot be bound only by a compression force. The binder may be present in the pharmaceutical composition in the form of a single compound or in the form of a mixture of compounds. Examples of binding agents include starch, pregelatinized starch (PGS), cellulose, microcrystalline cellulose, silicified microcrystalline cellulose, methyl cellulose, ethyl cellulose, hydroxypropyl methyl cellulose (HPMC), hydroxypropyl cellulose (HPC), sodium carboxy methyl cellulose, polyvinyl pyrrolidone (PVP), polyethylene glycol (PEG), polyvinyl alcohol/polyethylene glycol graft copolymer, polyvinyl alcohols, polymethacrylates, polyvinylcaprolactam, vinylpyrrolidone-vinyl acetate copolymers, and/or mixtures or derivatives thereof. Preferred binder according to the present invention is hydroxypropyl cellulose (HPC). Preferably, the binder is present in an amount from about 0.5% wt. to about 2% wt. with respect to the core solid dosage form weight.

The term "diluent" as used herein is defined as an inert agent designed to increase the weight and/or the size of the pharmaceutical composition, for example in the case of a tablet. The diluent may be present in the pharmaceutical composition in the form of a single compound or in the form of a mixture of compounds. Examples of diluents include, but are not limited to lactose anhydrous, lactose monohydrate, maltose, microcrystalline cellulose, mannitol, sorbitol and/or a mixtures or derivatives thereof. Preferred diluent according to the present invention is lactose monohydrate and/or microcrystalline cellulose and/or a mixture thereof. Lactose monohydrate is present in an amount from about 30% wt. to about 40% wt., preferably, in an amount from about 30% wt. to about 35% wt. with respect to the core solid dosage form weight. Microcrystalline cellulose is present in an amount from about 10% wt. to about 20% wt., preferably, in an amount from about 10% wt. to about 15% wt. with respect to the core solid dosage form weight.

The term "disintegrant" as used herein is defined as an accelerating agent of the disintegration of a solid oral dosage form and the dispersion of the active ingredient in water or gastrointestinal fluids. The disintegrant may be present in the pharmaceutical composition in the form of a single compound or in the form of a mixture of compounds. Examples of disintegrants include, but are not limited to croscarmellose sodium, carboxymethyl cellulose calcium, crospovidone, potassium, sodium starch glycolate, starch, pregelatinized starch and/or mixtures or derivatives thereof. Preferred disintegrant according to the present invention is croscarmellose sodium. Preferably, the disintegrant is present in an amount from about 1 to about 10% wt. with respect to the core solid dosage form weight. More preferably, the amount of disintegrant is from about 2% to about 5% wt. Most preferably, the amount of disintegrant is from about 4% wt. with respect to the core solid dosage form weight.

The term "lubricant" as used herein is defined as an agent able to decrease adhesion of a powder to punches and friction between particles. The lubricant may be present in the pharmaceutical composition in the form of a single compound or in the form of a mixture of compounds and may be present in the pharmaceutical composition as an intra-granular or extra-granular excipient, *i.e.,* lubricant. Preferably, the lubricant is present as an extra-granular lubricant. Examples of lubricants include, but are not limited to talc, magnesium stearate, stearic acid, zinc stearate, sodium stearyl fumarate, glyceryl dibehenate, hydrogenated castor oil, and/or mixtures or derivatives thereof. Preferred lubricant according to the present invention is magnesium stearate. Preferably, lubricant is present in an amount from about 0.1% to about 2% wt. with respect to the core solid dosage form weight. More preferably, the amount of lubricant is from about 0.5% to 1.5% wt., most preferably, the amount of lubricant is about 0.7% wt., with respect to the core solid dosage form weight.

The term "surfactant" or "emulsifier" as used herein is defined as a surface-active agent able to solubilize poorly soluble drugs, improve their dissolution profile, promote permeation, improve drug delivery, and/or enhance stabilization. The surfactant may be present in the pharmaceutical composition in the form of a single compound or in the form of a

mixture of compounds. Examples of surfactants include, but are not limited to, sodium lauryl sulfate, polyoxyethylene sorbitan fatty acid esters (polysorbates) and/or mixtures or derivatives thereof. Preferred surfactant according to the present invention is sodium lauryl sulfate. Surfactant is present in an amount from about 0.1% to about 2% wt., preferably, the amount of surfactant is from about 0.1% to 1.0% wt., most preferably, the amount of surfactant is about 0.3% wt., with respect to the core solid dosage form weight.

The term "around" or "about" is used herein to mean approximately, roughly, or within a certain region of a specific value or a range, but not strictly and doctrinally limited to said value or a range, including the error margins as well. When the term "around" or "about" or similar terms as mentioned in this paragraph are used in conjunction with a numerical range or a value, it modifies that range by extending the boundaries both above and below the numerical values set by that range or a value. In general, this term is used herein to modify a numerical range or a value above and below the stated value by a variance of at least 10%. The term "% wt." or "% by weight" is used herein to mean the percentage by weight of each ingredient in the uncoated or a coated solid oral dosage form, including or excluding any coatings.

The term "pharmaceutical composition", "pharmaceutical formulation" and/or "formulation" is used herein to mean a pharmaceutical composition comprising a therapeutically effective amount of an active pharmaceutical ingredient and one or more pharmaceutically acceptable excipients, provided as a final dosage form in form of a tablet, capsuled, granulate or others. The excipients can serve various roles such as aiding in the stability, bioavailability, and other properties of the pharmaceutical composition. The preferred pharmaceutical composition according to the present invention is a fixed-dose pharmaceutical composition comprising two active pharmaceutical ingredients and one or more pharmaceutically acceptable excipients. Even more preferably, said pharmaceutical composition according to the present invention is provided in a form of a one-layer solid oral dosage form.

The term "one-layer solid oral dosage form" is used herein to mean that the pharmaceutical composition, as described herein, preferably in a form of a tablet, is formed by a one single layer that contains both active ingredients Ambrisentan and Tadalafil and further along with one or more pharmaceutically acceptable excipients, wherein the active ingredients Ambrisentan and Tadalafil are not present in said pharmaceutical composition in separate layers. The active ingredients Ambrisentan and Tadalafil along with one or more pharmaceutically acceptable excipients comprised in that one single layer may be present in separate granules or in any other form therein, and during the manufacturing process are compressed together to form said one-layer solid oral dosage form. The one-layer solid oral dosage form may optionally be coated by one or more coating layers, preferably a film coating layer, wherein the one or more coating layers is or is not comprising said active ingredients Ambrisentan and Tadalafil.

## Examples

The following examples are illustrating some of the embodiments of the present invention without however limiting the scope of the invention, which is defined accordingly by the claims.

### Comparative example 1 - Ambrisentan formulation example (Volibris)

| Material | Function | Formula (mg/tablet) | Ratio (%) |
|---|---|---|---|
| **Ambrisentan tablet core:** | | | |
| Ambrisentan | API | 10.00 | 7.14 |
| Lactose monohvdrate | Diluent | 90.00 | 64.29 |
| Microcrystalline cellulose | Diluent | 35.00 | 25.00 |
| Croscarmellose sodium | Disintegrant | 4.00 | 2.86 |
| Magnesium stearate | Lubricant | 1.00 | 0.71 |
| **Core Tablet Weight** | | **140.00** | **100.00** |
| **Film coating:** | | | |
| Opadry II red | | 7.00 | |
| **Total** | | **147.00** | |

The comparative example of the Ambrisentan pharmaceutical composition is the composition of the reference drug product Volibris 10mg, which is commercially available and was procured from the market.

**Comparative example 2 - Tadalafil formulation example (Adcirca)**

| Material | Function | Formula (mg/tablet) | Ratio (%) |
|---|---|---|---|
| **Tadalafil tablet core:** | | | |
| Tadalafil | API | 20.00 | 5.71 |
| Lactose monohvdrate | Diluent | 245.20 | 70.06 |
| Microcrystalline cellulose | Diluent | 52.50 | 15.00 |
| Croscarmellose sodium | Disintegrant | 22.40 | 6.40 |
| Hydroxypropyl cellulose (HPC) | Binder | 8.05 | 2.30 |
| Sodium lauryl sulfate | Surfactant | 0.98 | 0.28 |
| Magnesium stearate | Lubricant | 0.88 | 0.25 |
| **Core Tablet Weight** | | **350.00** | **100.00** |
| **Film coating:** | | | |
| Opadry II yellow | | 14.00 | |
| **Total** | | **364.00** | |

The comparative example of the Tadalafil pharmaceutical composition formulation is the composition of the reference drug product Adcirca 20mg, which is commercially available and was procured from the market.

**Formulation according to the invention**

**Example 1 - Formulation example of Ambrisentan 5 mg and Tadalafil 20 mg fixed-dose formulation**

| Material | Function | Formula (mg/tablet) | Ratio (%) |
|---|---|---|---|
| **Tadalafil granulate:** | | | |
| Tadalafil | API | 20.00 | 5.71 |
| Lactose monohydrate | Diluent | 121.00 | 34.57 |
| Croscarmellose sodium | Disintegrant | 15.00 | 4.29 |
| Sodium lauryl sulfate | Surfactant | 1.00 | 0.29 |
| Hydroxypropyl cellulose (HPC) | Binder | 3.00 | 0.86 |
| **Ambrisentan granulate:** | | | |
| Ambrisentan | API | 5.00 | 1.43 |
| Hydroxypropyl cellulose (HPC) | Binder | 6.00 | 1.71 |
| Lactose monohydrate | Diluent | 128.90 | 36.83 |
| Microcrystalline cellulose | Diluent | 47.50 | 13.57 |
| **Extra-granular phase:** | | | |
| Magnesium stearate | Lubricant | 2.60 | 0.74 |
| **Core Tablet Weight** | | **350.00** | **100.00** |
| Opadry TF Blue / Green / Red | | 10.50 | |
| Water | | q.s. | |

(continued)

| | | | |
|---|---|---|---|
| **Extra-granular phase:** | | | |
| **Total** | | **360.50** | |

The fixed-dose pharmaceutical composition example of Ambrisentan 5 mg and Tadalafil 20 mg was manufactured according to the process described below.

Manufacturing process

Preparation of Tadalafil granulate:

1) Sifting: Sifting lactose monohydrate and croscarmellose sodium and sieving through 1.0 mm;
2) Preparation of a granulation liquid: Dissolving hydroxypropyl cellulose (HPC) in water (850 rpm, 15 min) and adding sodium lauryl sulfate into the mixture until dissolved (650 rpm, 15 min), further adding Tadalafil into the mixture until dispersed and sieving the solution through 0.15 mm;
3) Granulation in a fluid bed dryer: Granulation is carried out by spraying a powder mixture obtained in step 1 with the prepared granulation liquid in step 2, drying is carried out at 60°C inlet air temperature until the loss on drying (LOD) is less than 2.0% and sieving through 1.2 mm.

Preparation of Ambrisentan granulate:

4) Sifting and mixing: Sifting Ambrisentan, lactose monohydrate, microcrystalline cellulose, and one half of the total hydroxypropyl cellulose (HPC) amount by sieving through 0.6 mm sieve and mixing it for 10 minutes;
5) Preparation of a granulation liquid: Dissolving the second half of the total hydroxypropyl cellulose (HPC) amount in water (900 rpm, 30 minutes);
6) Granulation: Granulation of the mixture obtained in step 4 with the granulation liquid prepared in step 5 and sieving of the wet granules through 5 mm (350 rpm, 7 minutes granulation time, 1 minute chopper 1500 rpm, solution rate 18 rpm);
7) Drying: Drying of the wet granules at 60°C until the loss on drying (LOD) is less than 2.0%;

Preparation of the fixed-dose composition

8) Mixing granules: Mixing of the prepared Tadalafil granulate and Ambrisentan granulate
9) Sifting and mixing: Sieving of magnesium stearate by 0.25 mm and adding it to the granules mix from step 8 and mixing it for 3 minutes.
10) Compression: Performing tablet compression of the resulting mixture obtained in step 9;
11) Optional coating: Optionally, performing a coating process.

**Example 2** - **Formulation example of Ambrisentan 5 mg and Tadalafil 40 mg fixed-dose formulation**

| **Material** | **Function** | **Formula (mg/tablet)** | **Ratio (%)** |
|---|---|---|---|
| **Tadalafil granulate:** | | | |
| Tadalafil | API | 40.00 | 10.81 |
| Lactose monohydrate | Diluent | 121.00 | 32.70 |
| Croscarmellose sodium | Disintegrant | 15.00 | 4.05 |
| Sodium lauryl sulfate | Surfactant | 1.00 | 0.27 |
| Hydroxypropyl cellulose (HPC) | Binder | 3.00 | 0.81 |

(continued)

| | | | |
|---|---|---|---|
| **Ambrisentan granulate:** | | | |
| Ambrisentan | API | 5.00 | 1.35 |
| Hydroxypropyl cellulose (HPC) | Binder | 6.00 | 1.62 |
| Lactose monohvdrate | Diluent | 128.90 | 34.84 |
| Microcrystalline cellulose | Diluent | 47.50 | 12.84 |
| **Extra-granular phase:** | | | |
| Magnesium stearate | Lubricant | 2.60 | 0.70 |
| **Core Tablet Weight** | | **370.00** | **100.00** |
| Opadry TF Blue / Green / Red | | 11.10 | |
| Water | | q.s. | |
| **Total** | | **381.10** | |

The fixed-dose pharmaceutical composition example of Ambrisentan 5 mg and Tadalafil 40 mg was manufactured according to the same manufacturing process as in example 1.

**Example 3 - Formulation example of Ambrisentan 10 mg and Tadalafil 40 mg fixed-dose formulation**

| **Material** | **Function** | **Formula (mg/tablet)** | **Ratio (%)** |
|---|---|---|---|
| **Tadalafil granulate:** | | | |
| Tadalafil | API | 40.00 | 10.81 |
| Lactose monohydrate | Diluent | 121.00 | 32.70 |
| Croscarmellose sodium | Disintegrant | 15.00 | 4.05 |
| Sodium lauryl sulfate | Surfactant | 1.00 | 0.27 |
| Hydroxypropyl cellulose (HPC) | Binder | 3.00 | 0.81 |
| **Ambrisentan granulate:** | | | |
| Ambrisentan | API | 10.00 | 2.70 |
| Hydroxypropyl cellulose (HPC) | Binder | 6.00 | 1.62 |
| Lactose monohydrate | Diluent | 123.90 | 33.49 |
| Microcrystalline cellulose | Diluent | 47.50 | 12.84 |
| **Extra-granular phase:** | | | |
| Magnesium stearate | Lubricant | 2.60 | 0.70 |
| **Core Tablet Weight** | | **370.00** | **100.00** |
| Opadry TF Blue / Green / Red | | 11.10 | |
| Water | | q.s. | |
| **Total** | | **381.10** | |

The fixed-dose pharmaceutical composition example of Ambrisentan 10 mg and Tadalafil 40 mg was manufactured according to the same manufacturing process as in example 1. Advantageously, the additional strengths 5/40 mg and 10/40mg are dose-proportional with the 5/20 mg strength from example 1, which further supports the easiness of the preparation process of the fixed-dose pharmaceutical composition according to the present invention.

**Example 4 - Formulation example of Ambrisentan 10 mg and Tadalafil 40 mg fixed-dose formulation**

The fixed-dose pharmaceutical composition example of Ambrisentan 10 mg and Tadalafil 40 mg according to the formulation example 3 was manufactured by an alternative process described below.

Manufacturing process

Preparation of Tadalafil granulate:

1) Sifting: Sifting Tadalafil, lactose monohydrate and croscarmellose sodium, sieving the mixture through 1.0 mm and mixing the mixture for 15 minutes;
2) Preparation of a granulation solution: Dissolving hydroxypropyl cellulose (HPC) and sodium lauryl sulfate in water until dissolved (850 rpm, 15 min);
3) High-shear mixer wet granulation: Granulation of the mixture obtained in step 1 with the granulation solution prepared in step 2 and sieving of the wet granules through 5 mm (350 rpm, 5 min granulation time, 30 rpm solution rate);

Preparation of Ambrisentan granulate:

4) Sifting and mixing: Sifting Ambrisentan, lactose monohydrate, microcrystalline cellulose, and one half of the total hydroxypropyl cellulose (HPC) amount by sieving through 0.6 mm sieve and mixing it for 10 minutes;
5) Preparation of a granulation solution: Dissolving the second half of the total hydroxypropyl cellulose (HPC) amount in water (900 rpm, 30 minutes);
6) Granulation: Granulation of the mixture obtained in Step 5 with the with the prepared granulation solution in step 5 and sieving of the wet granules through 5 mm (350 rpm, 7 minutes granulation time, 1 minute chopper 1500 rpm, solution rate 18 rpm);
7) Drying: Drying of the wet granules at 60 C until the loss on drying (LOD) is less than 2.0%;

Preparation of the fixed-dose composition

8) Mixing granules: Mixing of the prepared Tadalafil granulate and Ambrisentan granulate
9) Sifting and mixing: Sieving of magnesium stearate by 0.25 mm and adding it to the granules mix from step 8 and mixing it for 3 minutes.
10) Compression: Performing tablet compression of the resulting mixture obtained in step 9;
11) Optional coating: Optionally, performing a coating process.

**Example 5 - Dissolution and Disintegration Data**

The following results were obtained for the composition examples 1 and 4, when both compositions were prepared according to the manufacturing process of example 4, after 6 months (T6) at normal conditions (25°C ± 2°C and 65% RH ± 5% RH) and at accelerated conditions (40°C ± 2°C and 75% RH ± 5% RH).

| **Normal conditions at T6** | **5mg/20mg** | **10mg/40mg** |
|---|---|---|
| **Disintegration (min:sec)** | 1:40 | 2:48 |
| | 1:42 | 3:03 |
| | 1:43 | 3:10 |
| | 1:44 | 3:18 |
| | 1:48 | 3:21 |
| | 1:54 | 3:27 |

(continued)

| Normal conditions at T6 | 5mg/20mg | 10mg/40mg |
|---|---|---|
| Average disintegration (min:sec) | 1:45 | 3:11 |
| Tadalafil dissolution | 94.9% | 96.0% |
| Ambrisentan dissolution | 102.9% | 95.6% |
| Water at T0 | 4.3% | 4.2% |
| Water uptake at T6 | 4.6% | 4.3% |

| Accelerated conditions at T6 | 5mg/20mg | 10mg/40mg |
|---|---|---|
| Disintegration (min:sec) | 12:20 | 13:40 |
| | 12:28 | 13:53 |
| | 12:32 | 13:54 |
| | 12:46 | 14:40 |
| | 12:58 | 16:05 |
| | 13:05 | 17:38 |
| Average disintegration (min:sec) | 12:41 | 14:58 |
| Tadalafil dissolution | 97.1% | 62.6% |
| Ambrisentan dissolution | 60.9% | 91.8% |
| Water at T0 | 4.3% | 4.2% |
| Water uptake at T6 | 4.8% | 5.0% |

As discussed above, the inventors have surprisingly found that there is a significant disintegration time difference between the two stability conditions, and consequently, a significant drop in the dissolution times of both Ambrisentan (60.9% after 30 mins) and Tadalafil (62.6% after 30 mins) for the fixed-dose pharmaceutical composition of example 1 and 4, when prepared by the manufacturing process of example 4.

**Example 6** - **Hardness Testing**

The following results of hardness testing of composition examples 1 and 4, when both compositions were prepared according to the manufacturing process of example 4, were obtained after 6 months (T6) at normal conditions (25°C ± 2°C and 65% RH ± 5% RH) and at accelerated conditions (40°C ± 2°C and 75% RH ± 5% RH).

| Hardness | 5mg/20mg | 10mg/40mg |
|---|---|---|
| **Normal conditions at T6** | 116 N | 146 N |
| | 109 N | 124 N |
| | 107 N | 137 N |
| | **Average: 111 N** | **Average: 136 N** |
| **Accelerated conditions at T6** | 126 N | 149 N |
| | 135 N | 143 N |
| | 136 N | 143 N |
| | **Average: 132 N** | **Average: 145 N** |

As discussed above, the change in the dissolution properties and disintegration time was surprisingly not accompanied by a significant hardness change of the fixed-dose pharmaceutical composition of example 1 and 4, when prepared by the manufacturing process of example 4 after 6 months (T6) at normal conditions (25°C ± 2°C and 65% RH ±

5% RH) or at accelerated conditions (40°C ± 2°C and 75% RH ± 5% RH).

**Example 7 - Dissolution and Disintegration Data**

The following results were obtained for composition examples 1 and 3, when both compositions were prepared by the manufacturing process of example 1, after 3 months (T3) at normal conditions (25°C ± 2°C and 65% RH ± 5% RH) and at accelerated conditions (40°C ± 2°C and 75% RH ± 5% RH).

| **Normal conditions at T3** | **5mg/20mg** | **10mg/40mg** |
|---|---|---|
| **Disintegration (min:sec)** | 3:11 | 2:28 |
| | 3:15 | 2:35 |
| | 3:13 | 2:31 |
| | 3:10 | 2:31 |
| | 3:19 | 2:29 |
| | 3:23 | 2:30 |
| **Average disintegration (min:sec)** | 3:15 | 2:30 |
| **Tadalafil dissolution** | 100.3% | 97.4% |
| **Ambrisentan dissolution** | 101.4% | 103.9% |
| **Water at T0** | 4.5% | 4.5% |
| **Water uptake at T3** | 4.3% | 4.6% |
| **Accelerated conditions at T3** | **5mg/20mg** | **10mg/40mg** |
| **Disintegration (min:sec)** | 1:53 | 1:41 |
| | 1:43 | 1:30 |
| | 1:42 | 1:39 |
| | 1:48 | 1:34 |
| | 1:41 | 1:35 |
| | 1:43 | 1:32 |
| **Average disintegration (min:sec)** | 1:45 | 1:35 |
| **Tadalafil dissolution** | 101.4 % | 95.4 % |
| **Ambrisentan dissolution** | 100.3 % | 103.9 % |
| **Water at T0** | 4.5% | 4.5% |
| **Water uptake at T3** | 4.2% | 4.7% |

As discussed above, the present inventors have surprisingly found that there is a further significant advantage in utilizing the manufacturing process of fluid bed dryer granulation for achieving the preferred disintegration and dissolution time of Ambrisentan and Tadalafil in the fixed-dose pharmaceutical composition according to the present invention and prepared by the process described in example 1. The results obtained above show that the fixed-dose pharmaceutical composition prepared by a process comprising fluid-bed granulation of the Tadalafil granulate and high-shear mixer wet granulation of the Ambrisentan granulate provides said fixed-dose pharmaceutical composition with the preferred disintegration and dissolution time for both active ingredients Ambrisentan and Tadalafil after 3 months (T3) at normal conditions (25°C ± 2°C and 65% RH ± 5% RH) and also after 3 months (T3) at accelerated conditions (40°C ± 2°C and 75% RH ± 5% RH).

**Example 8 - Hardness Testing**

The following results of hardness testing for formulation examples 1 and 3 of Ambrisentan and Tadalafil fixed-dose pharmaceutical composition were obtained after 3 months (T3) at normal conditions (25°C ± 2°C and 65% RH ± 5%

RH) and at accelerated conditions (40°C ± 2°C and 75% RH ± 5% RH).

| Hardness | 5mg/20mg | 10mg/40mg |
|---|---|---|
| **Normal conditions at T3** | 96 N | 109 N |
| | 100 N | 106 N |
| | 95 N | 108 N |
| | **Average: 97** N | **Average: 108** N |
| **Accelerated conditions at T3** | 81 N | 83 N |
| | 83 N | 83 N |
| | 83 N | 85 N |
| | **Average: 82** N | **Average: 84** N |

This testing has confirmed that the process used for preparation of the fixed-dose pharmaceutical composition as described in example 1 has been able to maintain the required hardness properties after 3 months (T3) at normal conditions (25°C ± 2°C and 65% RH ± 5% RH) and also after 3 months (T3) at accelerated conditions (40°C ± 2°C and 75% RH ± 5% RH), while providing at the same time the desired dissolution properties and disintegration time for both active ingredients Ambrisentan and Tadalafil.

**Example 9** - Bioequivalence studies

Bioequivalence studies of the fixed-dose pharmaceutical composition of Ambrisentan and Tadalafil according to the present invention 5 mg / 20 mg (according to example 1) and 10 mg / 40 mg (according to example 3), were carried out in accordance with the Declaration of Helsinki, International Council for Harmonisation (ICH), Good Clinical Practice (GCP) guidelines, the EU Clinical Trials Directive 2001/20/EC and applicable regulatory guidelines.

The aim of this study was to compare the pharmacokinetic parameters (PK) of the fixed-dose pharmaceutical composition of Ambrisentan and Tadalafil according to the present invention with the reference drug products for each respective mono-product of Ambrisentan (Volibris) and Tadalafil (Adcirca).

Bioequivalence study design of Ambrisentan and Tadalafil 5 mg / 20 mg (according to example 1) (Test Product) and the reference drug products of each respective mono-product of 5 mg Ambrisentan and 20 mg Tadalafil (Reference Products): An open label, randomized, balanced, two-treatment, two-period, two-sequence, single-dose, crossover oral bioequivalence study comparing fixed dose combination of Ambrisentan and Tadalafil Tablets in healthy adult human male subjects (24 subjects) under fed condition. The results for each active ingredient of the fixed dose combination are provided in the table below.

Ambrisentan

| Pharmacokinetic Parameter | Test Product | Reference Product | Ratio [%] | 90% CI Lower [%] | 90% CI Upper [%] |
|---|---|---|---|---|---|
| $C_{max}$ (ng/ml) | 440.12 | 459.50 | 95.78 | 89.59 | 102.41 |
| $AUC_{0-t}$ (hr·ng/ml) | 4584.47 | 4587.29 | 99.94 | 97.35 | 102.59 |

Tadalafil

| Pharmacokinetic Parameter | Test Product | Reference Product | Ratio [%] | 90% CI Lower [%] | 90% CI Upper [%] |
|---|---|---|---|---|---|
| $C_{max}$ (ng/ml) | 404.42 | 383.90 | 105.34 | 96.05 | 115.54 |

(continued)

| Pharmacokinetic Parameter | Test Product | Reference Product | Ratio [%] | 90% CI Lower [%] | 90% CI Upper [%] |
|---|---|---|---|---|---|
| $AUC_{0-t}$(hr·ng/ml) | 13017.64 | 12128.16 | 107.33 | 101.71 | 113.21 |

Based on the above results, the 90% confidence interval (CI) of primary pharmacokinetic parameters $C_{max}$ and $AUC_{0-t}$ for the fixed-dose pharmaceutical composition of Ambrisentan and Tadalafil according to the present invention 5 mg / 20 mg (Test Product) and the reference drug products for each respective mono-product of 5mg Ambrisentan and 20 mg Tadalafil (Reference Product) are within the acceptance limits of 80.00%-125.00% and hence it can be concluded that the fixed-dose pharmaceutical composition according to the present invention is bioequivalent with the respective reference drug mono-products, as it demonstrated a comparable extent and rate of absorption of Ambrisentan and Tadalafil to the respective mono-products of Ambrisentan and Tadalafil.

Bioequivalence study design of Ambrisentan and Tadalafil 10 mg / 40 mg (according to example 3) (Test Product) and the reference drug products for each respective mono-product of 10 mg Ambrisentan and 2 × 20 mg Tadalafil (Reference Product): An open label, randomized, balanced, two-treatment, two-period, two-sequence, single-dose, crossover oral bioequivalence study comparing fixed dose combination of Ambrisentan and Tadalafil Tablets in healthy adult human male subjects (24 subjects) under fasting condition. The results for each active ingredient of the fixed dose combination are provided in the table below.

Ambrisentan

| Pharmacokinetic Parameter | Test Product | Reference Product | Ratio [%] | 90% CI Lower [%] | 90% CI Upper [%] |
|---|---|---|---|---|---|
| $C_{max}$ (ng/ml) | 1167.475 | 1066.545 | 109.46 | 99.85 | 120.00 |
| $AUC_{0-t}$ (hr-ng/ml) | 9350.272 | 9318.699 | 100.34 | 96.59 | 104.23 |

Tadalafil

| Pharmacokinetic Parameter | Test Product | Reference Product | Ratio [%] | 90% CI Lower [%] | 90% CI Upper [%] |
|---|---|---|---|---|---|
| $C_{max}$ (ng/ml) | 542.451 | 596.900 | 90.88 | 83.40 | 99.03 |
| $AUC_{0-t}$ (hr-ng/ml) | 19624.888 | 21497.127 | 91.29 | 84.33 | 98.82 |

Based on the above results, the 90% confidence interval (CI) of primary pharmacokinetic parameters $C_{max}$ and $AUC_{0-t}$ for the fixed-dose pharmaceutical composition of Ambrisentan and Tadalafil according to the present invention 10 mg / 40 mg (Test Product) and the reference drug products for each respective mono-product of 10 mg Ambrisentan and 2 × 20 mg Tadalafil (Reference Product) are within the acceptance limits of 80.00%-125.00% and hence it can be concluded that the fixed-dose pharmaceutical composition according to the present invention is bioequivalent with the respective reference drug mono-products, as it demonstrated a comparable extent and rate of absorption of Ambrisentan and Tadalafil to the respective mono-products of Ambrisentan and Tadalafil.

The "$C_{max}$" (geometric mean value) refers to the maximum concentration that a drug achieves in the bloodstream after administration, before it starts to decline. It provides information about the peak exposure of the drug to the body.

The "$AUC_{0-t}$" refers to the total exposure of the drug in the body from the time of administration (0, time zero) until the last measurable concentration (time t). In the present application, the "$AUC_{0-t}$" (geometric mean value) refers to the area under the plasma concentration-time curve from 0 hours to the 72 hours concentration or the last sampling time t, whichever occurs first. It is calculated by plotting the drug concentration in the blood against time and measuring the area under the resulting curve. The "$AUC_{0-t}$" is used to quantify the drug's absorption and overall bioavailability.

The "Ratio" (geometric mean value) usually refers to the percentage comparison of pharmacokinetic parameters (such as $C_{max}$ or $AUC_{0-t}$) between the test product and the reference product. It is often expressed as the ratio of the

geometric means of these parameters.

**Example 10** - Particle Size Distribution (PSD) of Ambrisentan

The particle size distribution of Ambrisentan used in the reference drug product Volibris 10 mg (lot no. CFHVZ - composition according to comparative example 1) was measured using hot stage microscopy. The obtained particle size distribution of said sample was the following:

| | **Particle Size Distribution (μm)** |
|---|---|
| d (10) | 2.2 |
| d (50) | 3.7 |
| d (90) | 7.0 |

Based on the above reverse engineering particle size study of the reference drug product Volibris, the particle size of Ambrisentan active ingredient used in the formulation examples according to the present invention, was similarly micronized as the particle size distribution of Ambrisentan as used in the reference drug product Volibris.

**Example 11 -** Particle Size Distribution (PSD) of Tadalafil

The particle size distribution of Tadalafil used in the reference drug product Adcirca 20 mg (lot no. D442939 - composition according to comparative example 2) was measured using hot stage microscopy. The obtained particle size distribution of said sample was the following:

| | **Particle Size Distribution (μm)** |
|---|---|
| d (10) | 2.0 |
| d (50) | 3.0 |
| d (90) | 4.8 |

Based on the above reverse engineering particle size study of the reference drug product Adcirca, the particle size of Tadalafil active ingredient used in the formulation examples according to the present invention, was similarly micronized according to the particle size distribution of Tadalafil as used in the reference drug product Adcirca.

**<u>List of used analytical methods</u>**

**Dissolution studies**

**Tadalafil Dissolution**

Measured according to European Pharmacopeia 11.0, monograph 2.9.3. (Ph. Eur. 2.9.3.) and liquid chromatography according to European Pharmacopeia 11.1, monograph 2.2.29. (Ph. Eur. 2.2.29.).

Chromatographic Conditions:

| | |
|---|---|
| Instrument | : HPLC |
| Dedector | : UV-VIS, PDA |
| Column | : AGILENT Eclipse XDB-C8 (150 mm x 4.6 mm, 5 μm) |
| Wavelength | : 215 nm / 215nm |
| Flow Rate | : 1.0 mL/dk / 1.0 mL/dk |
| Injection Volume | : 20 μL / 20 μL |
| Analysis Time | : 12 dk / 12 min. |

(continued)

| | |
|---|---|
| Sample Temperature | : 25°C / 25°C |
| Column Temperature | : 40°C / 40°C |

| | |
|---|---|
| Blank Solution: | Diluent |
| Diluent: | Dissolution media |
| Mobile Phase: | Prepared by mixing the given amounts (pH 3.5 buffer solution: Acetonitrile: Trifluoroacetic Acid) (55:45:0.1) (v/v/v) and degassed in an ultrasonic bath for 2-3 minutes. |

Preparation of pH 3.5 Buffer Solution: 17.8 g disodium hydrogen phosphate dehydrate which is equivalent to the 14.2 g disodium hydrogen phosphate is transferred to the 2000 mL of volumetric flask, add some water and dissolved by stirring and make up to volume with water. pH is adjusted to the 3.5 ±0.5 with 85% ortho-phosphoric acid.

Dissolution Conditions:

| | |
|---|---|
| Apparatus | : Paddle |
| Dissolution Media | : 0.4% Sodium Lauryl Sulfate Solution |
| Speed | : 50 rpm |
| Dissolution Time | : 30 minutes |
| Temperature | : 37°C ± 0.5°C |
| Filter | : 35 μm Full-Flow Filter |
| Volume | : 1000 mL |
| Diluent | : Dissolution media |

Preparation of 0.4% Sodium Dodecyl Sulfate (0.4% SLS) Dissolution Media: Weigh 40.0 gr sodium dodecyl sulfate into 10 L flask which is contained 2 L deionized water and mix with a magnetic stirrer for an hour. Add 2 L of deionized water and stir for 15 minutes. Add 6 L of deionized water and stir 10 minutes.

Preparation of Stock Tadalafil Standard Solution: Approximately 40.0 mg of the Tadalafil working standard is transferred to a 100.0 mL volumetric flask, and 30 mL of solvent is added. It is kept in an ultrasonic bath for 15 minutes, shaken every 2-3 minutes to ensure dissolution. After reaching room temperature, it is brought to volume with the solvent. ($C_{Tadalafil}$= 0.40 mg/mL).

Preparation of Standard Solution: 5.0 mL of Stock Tadalafil standard solution is taken and transferred to a 50.0 mL volumetric flask and the volume is completed with 0.4% SLS, it is degassed in an ultrasonic bath for 15 minutes. It is filtered through a 0.45 μm PTFE filter and transferred to an HPLC vial. ($C_{Tadalafil}$= 0.04mg/mL)

One control standard solution is prepared identically with the standard solution.

Preparation of Sample Solution: 1000 mL dissolution media is placed into each dissolution vessel. Dissolution media is brought to 37°C ± 0.5 °C. Ambrisentan Tadalafil 5mg/20mg tablet 6 tablets are weighed individually, and they are placed into the vessel at the same time. At the end of 30 minutes, samples are taken from each vessel by using 35 μm full flow filter into HPLC vial. ($C_{Tadalafil}$= 0. 04 mg/mL).

Procedure: Condition the column up to get a smooth baseline, for at least 30 minutes. Inject Standard solution 6 consecutive times. Inject Control standard solution 2 times. 6 parallel samples are prepared, each sample is injected once. Inject standard solution 2 times at the end of the serial or 6 sample injections. The similarity factor between two standard preparations should be between 98.0 % - 102.0%.

System Suitability Test: Suitability of standard solution injections are controlled for following parameters. RSD between Tadalafil peaks areas obtained from 6 consecutive standard injections should be ≤ 2.0 %. The theoretical plate count of Tadalafil peak obtained from standard preparation should be ≥ 2000. The symmetry factor of Tadalafil peak obtained from standard preparation should be not more than 2.0. The similarity factor between two standard preparations should be between 98.0 % -102.0%.

Calculations

Standard Solution Concentration:

$$C_{Std} = \frac{Wstd}{V} \times P \times \frac{1}{S}$$

$C_{Std}$ : Tadalafil standard solution concentration (mg/mL)
$W_{Std}$ : Weigh of Tadalafil working standard (mg)
V : Dilution volume (100mL)
P : Potency of working standard
S : Dilution concentration (10)

Dissolution:

$$\text{Tadalafil (\%)} = \left[\frac{A_T}{A_{std}}\right] \times \left[\frac{C_{std}}{1/V}\right] x \frac{100}{ED(LC)}$$

$A_{Test}$ : Peak area of Tadalafil obtained to injection of test solution.
$A_{Std}$ : Peak area of Tadalafil obtained to injection of standard solution.
Csta : Calculated Tadalafil standard solution concentration (mg/mL)
V : Dilution factor (1000 mL)
ED/LC : Label claim

**Ambrisentan Dissolution**

Measured according to European Pharmacopeia 11.0, monograph 2.9.3. (Ph. Eur. 2.9.3.) and liquid chromatography according to European Pharmacopeia 11.1, monograph 2.2.29. (Ph. Eur. 2.2.29.).

Chromatographic Conditions:

| | |
|---|---|
| Instrument | : HPLC |
| Dedector | : UV-VIS, PDA |
| Column | : AGILENT Eclipse XDB-C8 (150 mm × 4.6 mm, 5μm) |
| Wavelength | : 215 nm / 215nm |
| Flow Rate | : 1.0 mL/dk / 1.0 mL/dk |
| Injection Volume | : 20 μL / 20 μL |
| Analysis Time | : 12 dk / 12 min. |
| Sample Temperature | : 25°C / 25°C |
| Column Temperature | : 40°C / 40°C |

Blank Solution: Diluent
Diluent: Dissolution media
Mobile Phase: Prepared by mixing the given amounts (pH 3.5 buffer solution: Acetonitrile: Trifluoroacetic Acid) (55:45:0.1) (v/v/v) and degassed in an ultrasonic bath for 2-3 minutes.

Preparation of pH 3.5 Buffer Solution: 17.8 g disodium hydrogen phosphate dehydrate which is equivalent to the 14.2 g disodium hydrogen phosphate is transferred to the 2000 mL of volumetric flask, add some water and dissolved by stirring and make up to volume with water. pH is adjusted to the 3.5 ±0.5 with 85% ortho-phosphoric acid.

Dissolution Conditions:

| | |
|---|---|
| Apparatus | : Paddle |
| Dissolution Media | : pH:5.0 Buffer Solution |
| Speed | : 60 rpm |
| Dissolution Time | : 30 minutes |
| Temperature | : 37°C ± 0.5°C |
| Filter | : 70 μm Full-Flow Filter |
| Volume | : 900 mL |
| Diluent | : Dissolution media |

Preparation of pH 5.0 Buffer Solution: 4.0 g Sodium Acetate is weighed into 1L flask. 1L of distilled water is added, and then 1mL of Acetic Acid is introduced. The mixture is stirred on a magnetic stirrer for 1 hour. pH is adjusted to 5.0 ± 0.05 using diluted Acetic Acid or diluted Sodium Hydroxide solution.

Preparation of Stock Ambrisentan Standard Solution: Approximately 11.0 mg of the Ambrisentan working standard is transferred to a 100.0 mL volumetric flask, and 30 mL of solvent is added. It is kept in an ultrasonic bath for 15 minutes, shaken every 2-3 minutes to ensure dissolution. After reaching room temperature, it is brought to volume with the solvent. ($C_{Ambrisentan}$= 0.11 mg/mL)

Preparation of Standard Solution: 5.0 mL of Stock Ambrisentan standard solution is taken and transferred to a 50.0 mL volumetric flask and the volume is completed with pH 5.0 buffer, It is degassed in an ultrasonic bath for 15 minutes. It is filtered through a 0.45 μm PTFE filter and transferred to an HPLC vial. ($C_{Ambrisentan}$= 0.011 mg/mL)

One control standard solution is prepared identically with the standard solution.

Preparation of Sample Solution: 900 mL dissolution media is placed into each dissolution vessel. Dissolution media is brought to 37°C ± 0.5 °C. Ambrisentan Tadalafil 5mg/20mg tablet 6 tablets are weighed individually, and they are placed into the vessel at the same time. At the end of 30 minutes, samples are taken from each vessel by using 70 μm full flow filter into HPLC vial. ($C_{Ambrisentan}$= 0. 011 mg/mL).

Procedure: Condition the column up to get a smooth baseline, for at least 30 minutes. Inject Standard solution 6 consecutive times. Inject Control standard solution 2 times. 6 parallel samples are prepared, each sample is injected once. Inject standard solution 2 times at the end of the serial or 6 sample injections. The similarity factor between two standard preparations should be between 98.0 % - 102.0%.

System Suitability Test: Suitability of standard solution injections are controlled for following parameters. RSD between Ambrisentan peaks areas obtained from 6 consecutive standard injections should be ≤ 2.0 %. The theoretical plate count of Ambrisentan peak obtained from standard preparation should be ≥ 2000. The symmetry factor of Ambrisentan peak obtained from standard preparation should be not more than 2.0. The similarity factor between two standard preparations should be between 98.0 % -102.0%.

Calculations

Standard Solution Concentration:

$$C_{Std} = \frac{Wstd}{V} \times P \times \frac{1}{S}$$

$C_{Std}$ : Standard solution concentration (mg/mL)
$W_{Std}$ : Weigh of standard (mg)
V : Dilution volume (100mL)
P : Potency of working standard
S : Dilution concentration (10)

Dissolution:

$$\text{Ambrisentan (\%)} = \left[\frac{A_T}{A_{std}}\right] \times \left[\frac{C_{std}}{1/V}\right] x \frac{100}{ED(LC)}$$

$A_{Test}$ : Peak area of Ambrisentan obtained to injection of test solution.
$A_{Std}$ : Peak area of Ambrisentan obtained to injection of standard solution.
$C_{Std}$ : Calculated Ambrisentan standard solution concentration (mg/mL)
V : Dilution factor (900 mL)
ED/LC : Label claim

**Disintegration studies**

Measured according to European Pharmacopeia 11.0, monograph 2.9.1. (Ph. Eur. 2.9.1.) - Test A, in distilled water without using the disc.

**Hardness testing**

Measured according to European Pharmacopeia 11.0, monograph 2.9.8. (Ph. Eur. 2.9.8.).

Hardness of 10 core tablets was measured by using a suitable hardness measuring device according to Ph. Eur. 2.9.8. and the average hardness was calculated.

**Water uptake testing**

Measured according to European Pharmacopeia 11.0, monograph 2.5.32. (Ph. Eur. 2.5.32.), with a 0.5 g sample by using Karl Fischer device.

**Particle Size Distribution (PSD)**

Particle Size Distribution (PSD) was measured using hot stage microscopy.

Preparation of Sample: The tablet contents were dispersed in water (approx. 1:5 w/v ratio) and vortexed for 2 min to dissolve the soluble contents in water. The mixture was centrifuged for 1 min at 5000 rpm. The supernatant containing soluble ingredients was discarded. Sample from the sediment was mounted on glass slide and observed under microscope for particle size analysis. The API and tablet samples were subjected to similar sample preparation and analyzed.

Hot stage microscopy: Particle size of API in the tablets was determined with a pre-calibrated stage micrometer using optical/polarized hot stage microscope (Leica DMLP, Leica, Germany) equipped with a controlled heating and cooling stage (LTS350, Linkam) and an imaging system (VTO 232, JVC - Digital camera and Linksys 32 imaging software, Linkam, England). A small amount of sample was mounted on glass slide and observed under the microscope. Sample was observed at 500X magnification with a least count of 3.5 micron.

Identification of API: For particle size analysis of API, drug particles were differentiated from other ingredients present in tablets through microscopy. This was done based on difference in the melting point of the drug and other ingredients. API particles could be differentiated by hot-stage microscopy. Particle size of above 500 particles was determined and data was grouped into class intervals.

**Claims**

1. A fixed-dose pharmaceutical composition comprising Ambrisentan or its pharmaceutically acceptable salt, ester, solvate, polymorph, enantiomer or mixture thereof and Tadalafil or its pharmaceutically acceptable salt, ester, solvate, polymorph, enantiomer or mixture thereof, and one or more pharmaceutically acceptable excipients, wherein the pharmaceutical composition is in a form of a one-layer solid oral dosage form and comprises Ambrisentan granulate and Tadalafil granulate.

2. The fixed-dose pharmaceutical composition according to claim 1, wherein the Ambrisentan granulate is free from any lubricant and/or any disintegrant.

3. The fixed-dose pharmaceutical composition according to claim 1 and/or 2, wherein the Ambrisentan granulate comprises excipients selected from a diluent, a binder and/or a mixture thereof.

**4.** The fixed-dose pharmaceutical composition according to any of the preceding claims, wherein the Tadalafil granulate is free from any lubricant.

**5.** The fixed-dose pharmaceutical composition according to any of the preceding claims, wherein the Tadalafil granulate comprises excipients selected from a diluent, a disintegrant, a binder, a surfactant and/or a mixture thereof.

**6.** The fixed-dose pharmaceutical composition according to any of the preceding claims, wherein a lubricant is present as an extra-granular excipient.

**7.** The fixed-dose pharmaceutical composition according to any of the preceding claims, wherein the Tadalafil granulate is prepared by a fluid-bed granulation process and/or the Ambrisentan granulate is prepared by a high-shear mixer wet granulation process.

**8.** The fixed-dose pharmaceutical composition according to any of the preceding claims, wherein the Ambrisentan granulate and the Tadalafil granulate is in a weight ratio from about 1:0.5 to about 1:1.5, preferably from about 1:0.7 to about 1:1, more preferably from about 1:0.8 to about 1:0.9.

**9.** The fixed-dose pharmaceutical composition according to any of the preceding claims, wherein after 3 months at accelerated conditions at 40°C ± 2°C and 75% RH ± 5% RH, the disintegration time of the fixed-dose pharmaceutical composition is less than 5 minutes, preferably less than 4 minutes, as measured by European Pharmacopeia 11.0, monograph 2.9.1. - Test A, in distilled water without using the disc.

**10.** The fixed-dose pharmaceutical composition according to any of the preceding claims, wherein after 3 months at accelerated conditions at 40°C ± 2°C and 75% RH ± 5% RH, the fixed-dose pharmaceutical composition has a dissolution profile such that within the first 30 minutes, preferably within the first 15 minutes, more than 85% of Ambrisentan, or its pharmaceutically acceptable salt, ester, solvate, polymorph, enantiomer or mixture thereof, is released, as measured by European Pharmacopeia 11.0, monograph 2.9.3., dissolution Apparatus 2 in 900 ml of pH 5.0 acetate buffer solution at 37°C ± 0.5°C with the paddle speed of 60 rpm, and/or such that within the first 30 minutes, preferably within the first 15 minutes, more than 85% of Tadalafil, or its pharmaceutically acceptable salt, ester, solvate, polymorph, enantiomer or mixture thereof, is released is released, as measured by as measured by European Pharmacopeia 11.0, monograph 2.9.3., dissolution Apparatus 2 in 1000 ml of 0.4% sodium lauryl sulfate solution at 37°C ± 0.5°C with the paddle speed of 50 rpm.

**11.** The fixed-dose pharmaceutical composition according to any of the preceding claims, wherein the Ambrisentan granulate comprises:

a) from about 1% wt. to about 5% wt. of Ambrisentan, or its pharmaceutically acceptable salt, ester, solvate, polymorph, enantiomer or mixture thereof, with respect to the core solid dosage form weight;
b) from about 0.5% wt. to about 2% wt. of binder with respect to the core solid dosage form weight, where preferably the binder is selected from the group comprising pregelatinized starch (PGS), cellulose, microcrystalline cellulose, silicified microcrystalline cellulose, methyl cellulose, ethyl cellulose, hydroxypropyl methyl cellulose (HPMC), hydroxypropyl cellulose (HPC), sodium carboxy methyl cellulose, polyvinyl pyrrolidone (PVP), polyethylene glycol (PEG), polyvinyl alcohol/polyethylene glycol graft copolymer, polyvinyl alcohols, polymethacrylates, polyvinylcaprolactam, vinylpyrrolidone-vinyl acetate copolymers, and/or mixtures or derivatives thereof;
c) from about 40% wt. to about 55% wt. of diluent with respect to the core solid dosage form weight, where preferably the diluent is selected from the group comprising lactose anhydrous, lactose monohydrate, maltose, microcrystalline cellulose, mannitol, sorbitol and/or mixtures or derivatives thereof;

and wherein the Tadalafil granulate comprises:

a) from about 5% wt. to about 15% wt. of Tadalafil, or its pharmaceutically acceptable salt, ester, solvate, polymorph, enantiomer or mixture thereof, with respect to the core solid dosage form weight;
b) from about 30% wt. to about 35% wt. of diluent with respect to the core solid dosage form weight, where preferably the diluent is selected from the group comprising lactose anhydrous, lactose monohydrate, maltose, microcrystalline cellulose, mannitol, sorbitol and/or mixtures or derivatives thereof;
c) from about 0.5% wt. to about 2% wt. of binder with respect to the core solid dosage form weight, where preferably the binder is selected from the group comprising pregelatinized starch (PGS), cellulose, microcrystalline cellulose, silicified microcrystalline cellulose, methyl cellulose, ethyl cellulose, hydroxypropyl methyl cellulose (HPMC), hydroxypropyl cellulose (HPC), sodium carboxy methyl cellulose, polyvinyl pyrrolidone (PVP), polyethylene glycol (PEG), polyvinyl alcohol/polyethylene glycol graft copolymer, polyvinyl alcohols, polymethacrylates, polyvinylcaprolactam, vinylpyrrolidone-vinyl acetate copolymers, and/or mixtures or derivatives thereof;
d) from about 1 to about 10% wt. of disintegrant with respect to the core solid dosage form weight, where preferably the disintegrant is selected from the group comprising croscarmellose sodium, carboxymethyl cellulose calcium, crospovidone, potassium, sodium starch glycolate, starch, pregelatinized starch and/or mixtures or derivatives thereof;
e) from about 0.1% to about 2% wt. of surfactant with respect to the core solid dosage form weight, where preferably the surfactant is selected from the group comprising sodium lauryl sulfate, polyoxyethylene sorbitan fatty acid esters (polysorbates) and/or mixtures or derivatives thereof.

**12.** The fixed-dose pharmaceutical composition according to claim 11, wherein the Ambrisentan granulate comprises:

a) from about 1% wt. to about 5% wt. of Ambrisentan, with respect to the core solid dosage form weight;
b) from about 0.5% wt. to about 2% wt. of hydroxypropyl cellulose as the binder, with respect to the core solid dosage form weight;
c) from about 40% wt. to about 55% wt. of a mixture of lactose monohydrate and microcrystalline cellulose as the diluent, with respect to the core solid dosage form weight,

and wherein the Tadalafil granulate comprises:

a) from about 5% wt. to about 15% wt. of Tadalafil, with respect to the core solid dosage form weight;
b) from about 30% wt. to about 35% wt. of lactose monohydrate as the diluent, with respect to the core solid dosage form weight;
c) from about 0.5% wt. to about 2% wt. of hydroxypropyl cellulose as the binder, with respect to the core solid dosage form weight;
d) from about 1 to about 10% wt. of croscarmellose sodium as the disintegrant, with respect to the core solid dosage form weight;
e) from about 0.1% to about 2% wt. of sodium lauryl sulfate as the surfactant, with respect to the core solid dosage form weight.

**13.** The fixed-dose pharmaceutical composition according to any of the preceding claims, wherein

i) said pharmaceutical composition is comprising 5 mg Ambrisentan and 20 mg Tadalafil and wherein in human in vivo pharmacokinetic studies in which said pharmaceutical composition is administered in fed state, said pharmaceutical composition provides the following pharmacokinetic parameter selected from the group consisting of

- Ambrisentan maximum plasma concentration $C_{max}$ of about 440 ng/mL $\pm$ 10%,
- Ambrisentan area under the plasma concentration-time curve from 0 hours to the 72 hours $AUC_{0-t}$ of about 4584 hr-ng/ml $\pm$ 10%,
- Tadalafil maximum plasma concentration $C_{max}$ of about 404 ng/mL $\pm$ 10%,
- Tadalafil area under the plasma concentration-time curve from 0 hours to the 72 hours $AUC_{0-t}$ of about 13017 hr-ng/ml $\pm$ 10%;

and/or, wherein

ii) said pharmaceutical composition is comprising 10 mg Ambrisentan and 40 mg Tadalafil and wherein in human in vivo pharmacokinetic studies in which said pharmaceutical composition is administered in fasting state, said pharmaceutical composition provides the following pharmacokinetic parameter selected from the group consisting of:

- Ambrisentan maximum plasma concentration $C_{max}$ of about 1167 ng/mL $\pm$ 10%,
- Ambrisentan area under the plasma concentration-time curve from 0 hours to the 72 hours $AUC_{0-t}$ of about 9350 hr-ng/ml $\pm$ 10%,
- Tadalafil maximum plasma concentration $C_{max}$ of about 542 ng/mL $\pm$ 10%,
- Tadalafil area under the plasma concentration-time curve from 0 hours to the 72 hours $AUC_{0-t}$ of about 19624 hr-ng/ml $\pm$ 10%.

**14.** A process for preparation of the fixed-dose pharmaceutical composition according to any of the claims 1 to 13, wherein the Tadalafil granulate is prepared by a fluid-bed granulation process and wherein the Ambrisentan granulate is prepared by a high-shear mixer wet granulation process.

**15.** The process according to claim 14, further comprising mixing of the Tadalafil granulate and the Ambrisentan granulate, addition of an extra-granular lubricant, wherein preferably the extra granular lubricant is magnesium stearate, mixing the extra-granular lubricant with the Tadalafil granulate and the Ambrisentan granulate, and further compression of the mixture in order to form a one-layer solid oral dosage form.

Europäisches Patentamt
European Patent Office
Office européen des brevets

# EUROPEAN SEARCH REPORT

Application Number
EP 24 38 6091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/003678 A1 (XSPRAY MICROPARTICLES AB [SE]) 3 January 2014 (2014-01-03)<br>* page 23, lines 11-16; claims 1, 13, 23, 24 * | 4,5 | INV.<br>A61K9/20<br>A61K31/4985<br>A61K31/519 |
| A | WO 2011/030351 A2 (RUBICON RES PRIVATE LTD [IN]; PILGAONKAR PRATIBHA SUDHIR [IN] ET AL.) 17 March 2011 (2011-03-17)<br>* page 20, lines 23-25; claims 1, 4, 23-26 * | 1-15 | |
| X<br><br>Y | US 2012/269898 A1 (BELARDINELLI LUIZ [US] ET AL) 25 October 2012 (2012-10-25)<br>* lines 1-4, paragraph 0196 *<br>* lines 7-13, paragraph 0197 *<br>* paragraph [0029] *<br>* claims 15, 21, 22 * | 1-3,6-15<br><br>4,5 | |
| A | DATABASE EMBASE [Online]<br>ELSEVIER SCIENCE PUBLISHERS, AMSTERDAM, NL;<br>1 June 2019 (2019-06-01),<br>OKOUR M ET AL: "A Phase I Study to Show the Relative Bioavailability and Bioequivalence of Fixed-Dose Combinations of Ambrisentan and Tadalafil in Healthy Subjects",<br>XP002812739,<br>Database accession no. EMB-002001911813<br>* the whole document *<br>-/-- | 1-15 | TECHNICAL FIELDS SEARCHED (IPC)<br>A61K |

The present search report has been drawn up for all claims

| Place of search | Date of completion of the search | Examiner |
|---|---|---|
| The Hague | 12 December 2024 | Munzone, Alessia |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 38 6091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | & OKOUR MALEK ET AL: "A Phase I Study to Show the Relative Bioavailability and Bioequivalence of Fixed-Dose Combinations of Ambrisentan and Tadalafil in Healthy Subjects", CLINICAL THERAPEUTICS, vol. 41, no. 6, 1 June 2019 (2019-06-01), pages 1110-1127, XP093233203, AMSTERDAM, NL ISSN: 0149-2918, DOI: 10.1016/j.clinthera.2019.04.007 * section: Study Objective and Design; lines 12-25 - page 3 * ----- | 1-15 | |
| T | Anonymous: "What are the advantages of external lubrication VS blending lubricant? ¦ Pharmatec", , 23 September 2022 (2022-09-23), pages 1-3, XP093233365, Retrieved from the Internet: URL:https://pharmatec.be/events/ispray-what-are-the-advantages-of-external-lubrication-on-a-tablet-press-compared-to-folding-lubricant-into-the-blend/ * the whole document * & Pharmatec: "wayback", , 23 September 2021 (2021-09-23), pages 1-1, XP093233662, Retrieved from the Internet: URL:https://web.archive.org/ ----- | | TECHNICAL FIELDS SEARCHED (IPC) |

The present search report has been drawn up for all claims

| Place of search | Date of completion of the search | Examiner |
|---|---|---|
| The Hague | 12 December 2024 | Munzone, Alessia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

page 2 of 2

## ANNEX TO THE EUROPEAN SEARCH REPORT ON EUROPEAN PATENT APPLICATION NO. EP 24 38 6091

This annex lists the patent family members relating to the patent documents cited in the above-mentioned European search report. The members are as contained in the European Patent Office EDP file on
The European Patent Office is in no way liable for these particulars which are merely given for the purpose of information.

12-12-2024

| Patent document cited in search report | Publication date | Patent family member(s) | Publication date |
|---|---|---|---|
| WO 2014003678 A1 | 03-01-2014 | NONE | |
| WO 2011030351 A2 | 17-03-2011 | NONE | |
| US 2012269898 A1 | 25-10-2012 | AR 083422 A1 | 21-02-2013 |
| | | AU 2011315891 A1 | 23-05-2013 |
| | | AU 2016204638 A1 | 04-08-2016 |
| | | BR 112013008983 A2 | 05-07-2016 |
| | | CA 2814518 A1 | 19-04-2012 |
| | | CN 103458690 A | 18-12-2013 |
| | | CY 1119007 T1 | 10-01-2018 |
| | | DK 2637664 T3 | 19-06-2017 |
| | | EA 201390428 A1 | 28-02-2014 |
| | | EA 201691759 A1 | 31-05-2017 |
| | | EP 2637664 A2 | 18-09-2013 |
| | | EP 3106163 A1 | 21-12-2016 |
| | | ES 2627944 T3 | 01-08-2017 |
| | | HR P20170848 T1 | 25-08-2017 |
| | | HU E033346 T2 | 28-11-2017 |
| | | JP 5806320 B2 | 10-11-2015 |
| | | JP 2014506870 A | 20-03-2014 |
| | | JP 2015178528 A | 08-10-2015 |
| | | KR 20130069855 A | 26-06-2013 |
| | | KR 20150002876 A | 07-01-2015 |
| | | LT 2637664 T | 10-11-2017 |
| | | MX 346730 B | 30-03-2017 |
| | | NZ 610012 A | 29-05-2015 |
| | | NZ 707213 A | 23-12-2016 |
| | | PL 2637664 T3 | 29-09-2017 |
| | | PT 2637664 T | 22-06-2017 |
| | | TW 201219372 A | 16-05-2012 |
| | | US 2012269898 A1 | 25-10-2012 |
| | | US 2014275098 A1 | 18-09-2014 |
| | | US 2016346280 A1 | 01-12-2016 |
| | | WO 2012051559 A2 | 19-04-2012 |
| | | ZA 201302746 B | 25-06-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9425442 A
- WO 9526716 A
- WO 9611914 A
- WO 9519978 A
- WO 9703985 A
- WO 0015228 A
- WO 2008073928 A
- WO 2012051559 A